# EUROPÄISCHE PATENTSCHRIFT

(11) **EP 1 490 019 B1**
(45) Veröffentlichungstag und Bekanntmachung des Hinweises auf die Patenterteilung: **08.06.2005**
(21) Anmeldenummer: 03745251.3
(22) Anmeldetag: 27.03.2003
(51) Int. Cl.: A61K 7/44

(54) **HAUTAUFHELLENDES KOSMETIKUM**
SKIN-BRIGHTENING COSMETIC
PRODUIT COSMETIQUE A EFFET ILLUMINATEUR

(30) Priorität: 28.03.2002 DE 10215342
(43) Veröffentlichungstag der Anmeldung: 29.12.2004
(73) Patentinhaber: Coty B.V., 2031 CC Haarlem (NL)
(72) Erfinder: GOLZ-BERNER, Karin, MC-98000 Monaco (MC); ZASTROW, Leonhard, MC-98000 Monaco (MC)
(74) Vertreter: Walter, Wolf-Jürgen
(86) Internationale Anmeldenummer: PCT/DE2003/001046
(87) Internationale Veröffentlichungsnummer: WO 2003/082238

(56) Entgegenhaltungen:
- WO-A-94/09756
- FR-A- 2 742 661
- DATABASE WPI Derwent Publications Ltd., London, GB; AN 1993-269739 XP002248310 N.N.: "Skin-whitening cosmetic material, preventing pigmentation by UV - contains oil-soluble glycyrrhiza extract, ascorbic acid (deriv.), kojic acid (deriv.), etc. and antiinflammatory agents" & JP 05 186324 A (SUNSTAR CHEM IND), 27. Juli 1993 (1993-07-27)
- N.N.: "Gatuline is an in vivo substantiated line of plant derived active ingredients" GATTEFOSSE - WITH IN VIVO SUBSTANTIATED CLAIMS, [Online] XP002248309 Gefunden im Internet: <URL:http://www.gattefosse.com/cosmetic/ma inviv2.htm> [gefunden am 2003-07-18]
- "Enzymolytic pearl liquor" CHEMICAL ABSTRACTS + INDEXES, AMERICAN CHEMICAL SOCIETY. COLUMBUS, US, Bd. 11, Nr. 124, 11. März 1996 (1996-03-11), Seite 1084 XP002017602 ISSN: 0009-2258
- PATENT ABSTRACTS OF JAPAN vol. 011, no. 124 (C-416), 17. April 1987 (1987-04-17) & JP 61 263905 A (POLA CHEM IND INC), 21. November 1986 (1986-11-21)
- KATSAMBAS A ET AL: "DISORDERS OF PIGMENTATION: UNAPPROVED TREATMENTS" CLINICS IN DERMATOLOGY, J.B. LIPPINCOTT, PHILADELPHIA, PA, US, Bd. 20, Nr. 6, 2002, Seiten 649-659, XP001148369 ISSN: 0738-081X

## Beschreibung

Die Erfindung betrifft ein Kosmetikum mit hautaufhellender Wirkung.

Es sind bereits hautaufhellende Mittel und Methoden bekannt, wie ein Verfahren zur Hautaufhellung mittels eines Gemisches aus einem antimikrobiellen Mittel, einer α- bzw. β-Hydroxysäure wie Glycolsäure oder Milchsäure bzw. Salicylsäure und einem pharmazeutisch annehmbaren Träger (WO 01/70189). Weiterhin ist aus WO 01/70190 ein hautaufhellendes (lightening) Mittel wie Niacinamid oder einem Precursor davon und Titaniumdioxid mit einer Teilchengröße von <100 nm bekannt. Die Wirkung dieser Mittel ist nicht immer befriedigend.

Der Erfindung liegt die Aufgabe zugrunde, ein neues hautaufhellendes kosmetisches Mittel bereitzustellen, das besonders gleichmäßig und über einen langen Zeitraum wirkt.

Erfindungsgemäß besteht das neue hautaufhellende Kosmetikum aus einem Gemisch von 0,01 bis 0,5 Gew-% Arbutin,
0,01 bis 0,05 Gew-% eines Perlenextraktes,
0,05 bis 0,3 Gew-% eines Extraktgemisches von Glycyrrhiza glabra und Aspergillusferment und
0,001 bis 0,1 Gew-% eines Extraktes von Astragalus,
sowie weiteren kosmetischen Hilfsstoffen, Trägerstoffen und Wirkstoffen bis 100 Gew-%, jeweils bezogen auf das Gesamtgewicht des Kosmetikums.

Arbutin als Wirkstoff der Bärentraube (Arctostaphylos) wirkt allgemein stark antibakteriell gegen Klebsiella- und Eschericha coli-Bakterien und wird als Antiseptikum insbesondere der Harnwege in der Pflanzenmedizin eingesetzt. Als Hydrochinonderivat kann es auch eine aufhellende Wirkung haben, die jedoch zum Teil sehr ungleichmäßig ist.

überraschenderweise zeigt eine Kombination von Arbutin mit anderen Pflanzenextrakten gemäß der erfindungsgemäßen Zusammensetzung eine besonders gleichmäßige Hautaufhellung ohne erkennbare Aufhellungsschwerpunkte und eine besonders langanhaltende Wirkung. Diese Wirkung war auch für den Fachmann nicht vorhersehbar, da z.B. Glycyrrhiza-Extrakte, insbesondere Glycyrrhiza glabra zwar für kortisonartige Wirkungen bekannt ist sowie bei Herpes und Gürtelrosen auch äußerlich angewendet werden kann, jedoch keinerlei Wirksamkeit in Bezug auf Hautaufhellungen zusammen mit anderen Extrakten beschrieben sind. Der Extrakt von Glycyrrhiza glabra ist ein wäßriger Extrakt der Wurzel.

Das gleiche gilt für Astragalus, der als Tragant (Astragalus gummifer) oder als Astragalus membranaceus oder A. complanatus eingesetzt werden kann, und bei dem äußerliche Wirkungen nicht detailliert beschrieben sind, sondern meist nur die immunsystemkräftigende und kreislaufanregende innere Wirkung. Der Extrakt ist ein wäßriger oder wäßrig-alkoholischer Extrakt. Astralagus ist kosmetisch auch als Verdickungsmittel bekannt. In den hier eingesetzten Konzentrationen von 0,001 bis 0,1 Gew-%, insbesondere 0,001 bis 0,05 Gew-%, besonders bevorzugt 0,001 bis 0,01 Gew-% ist diese Wirkung vernachlässigbar, und es zeigt sich eine unterstützende Wirkung in der Hautaufhellung.

Besonders wirksam erscheint das Gemisch von Glycyrrhiza-Extrakt mit einem Aspergillus-Ferment. Aspergillus, insbesondere Aspergillus niger als eine der bekanntesten Schimmelpilzarten, wird beispielsweise in der aeroben Zitronensäuregärung von Glucose eingesetzt. Bei dieser Fermentierung wird das Pilzmycel im Verlaufe des Verfahren von der Kulturbrühe abgetrennt, abgepreßt, getrocknet und meist als Viehfutter weiterverwendet. In einer speziell gereinigten Form kann auch eine kosmetische Weiterverwendung erfolgen. Das Verhältnis bei Gemischen beider Wirkstoffe kann im Bereich von 30:1 bis 5:1 liegen. Die hautaufhellende Wirkung ist noch stärker, wenn nicht die beiden Einzelsubstanzen separat sondern als Pre-Mix in die Formulierung eingebracht werden.

Das erfindungsgemäße Kosmetikum enthält weiterhin kosmetische Hilfs- und Trägerstoffe, wie sie üblicherweise in solchen Zubereitungen verwendet werden, z.B. Wasser, Konservierungsmittel, Farbstoffe, Verdickungsmittel, Duftstoffe, Alkohole, Polyole, Ester, Elektrolyte, Gelbildner, polare und unpolare Öle, Polymere, Copolymere, Emulgatoren, Stabilisatoren.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; Siliconöle oder Gemische zweier oder mehrerer davon.

Besonders geeignete Öle sind beispielsweise Mineralöle, Hydrogenated Polyisobuten, Polyisopren, Squalane, Tridecyltrimellitat, Trimethylpropan-triisostearat, Isodecylcitrat, Neopentylglycol-diheptanoat, PPG-15-stearylether, Cyclomethicone sowie pflanzliche Öle, wie Calendulaöl, Jojobaöl, Avocadoöl, Macadamianußöl, Rizinusöl, Weizenkeimöl, Traubenkernöl, Kukuinußöl, Distelöl, Nachtkerzenöl, Safloröl oder ein Gemisch mehrerer davon. Je nachdem welche Öle ausgewählt werden, werden die kosmetischen Eigenschaften beeinflußt, wie Transparenzgrad, Weichheit, Spreitungswirkung.

Als Ester oder Ether sind zum Beispiel geeignet Dipentaerythrityl hexacaprilate/hexacaprate/tridecyl trimellitate/tridecyl stearate/neopentyl glycol dicaprylate dicaprate, Propylene glycol dioctanoate 5, Propylene glycol dicaprylate 2,30 dicaprate, Tridecyl stearate/neopentyl glycol dicaprylate dicaprate/tridecyl trimellitate, Neopentyl glycol dioctanoate, Isopropyle myristate, Diisopropyl dimer dilinoleate, Trimethylpropane triisostearate, Myristyl ether, Stearyl ether, Cetearyl octanoate, Butyl ether, Dicaprylyl ether, PPG1-PEG9 Lauroyl glycol ether, PPG15 Stearyl ether, PPG14 Butyl ether, Fomblin HC25.

Einwertige höhere Alkohole wie Cetearylalkohol oder mehrwertige Alkohole (Polyole) sind ebenfalls mögliche Bestandteile des erfindungsgemäßen Kosmetikums. Dies sind z.B Propylenglycol, Dipropylenglycol, Ethylenglycol, Isoprenglycol, Glycerin, Sorbitol und Gemische davon. Der Anteil des Polyols liegt im Bereich von 0,1 to 40 Gew-%, vorzugsweise von etwa 5% bis etwa 20 Gew-% der Zusammensetzung.

Kosmetische Zusammensetzungen mit der erfindungsgemäßen Wirkstoffzubereitung können als O/W- oder W/O-Emulsionen vorliegen. Geeignete Emulgatoren für O/W- oder W/O-Emulsionen sind bekannt und sind beispielsweise Anlagerungsprodukte von Ethylenoxid an Fettsäuren, Ester oder Öle.

Die Zusammensetzung kann auch als Gel vorliegen. Zu kosmetischen geeigneten Gelbildnern gehören Carbomer, Xanthangummi, Carrageenan, Akaziengummi, Guargummi, Agar-Agar, Alginate und Tylosen, Carboxymethylcellulose, Hydroxyethylcellulose, quaternisierte Cellulose, quaternisierter Guar, bestimmte Polyacrylate wie Acrylates/C10-30 Alkyl Acrylate Crosspolymer, Polyvinylalkohol, Polyvinylpyrrolidon.

Das Kosmetikum kann weitere Wirkstoffe enthalten. Zu den kosmetischen Wirkstoffen gehören z. B. anorganische und organische Lichtschutzmittel, Radikalfänger, Feuchthaltemittel, Erweichungsmittel, Vitamine, Enzyme, pflanzliche Wirkstoffe, Polymere, Antioxidationsmittel, entzündungswidrige natürliche Wirkstoffe, mit Sauerstoff beladene asymmetrische lamellare Aggregate gemäß WO 94/00109; Aufschlußprodukte von Hefen oder pflanzlichen Stoffen, hergestellt durch ein schonendes Ultraschall-Aufschlußverfahren gemäß WO 94/13783, Kaolin sowie mit SiO₂ modifiziertes Kaolin gemäß WO94/17588.

Zu Antioxidationsmitteln gehören Vitamine wie Vitamin C und Derivate davon, beispielsweise Ascorbylacetate, -phosphate und -palmitate; Vitamin A und Derivate davon; Folsäure und deren Derivate, Vitamin E und deren Derivate, wie Tocopherylacetat; Flavone oder Flavonoide; Aminosäuren, wie Histidin, Glycin, Tyrosin, Tryptophan und Derivate davon; Harnsäure und Derivate davon.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isohexadecan, Isopropylisostearat, Stearinsäure, Isobutylpalmitat, Oleylalkohol, Isopropyllaurat, Decyloleat, Octadecan-2-ol, Isocetylalkohol, Cetylpalmitat, Siliconöle wie Dimethylpolysiloxan, Isopropylmyristat, Isopropylpalmitat, Polyethylenglycol, Lanolin, Kakaobutter, pflanzliche Öle wie Maisöl, Baumwollsamenöl, Olivenöl, mineralische Öle, Butylmyristat, Palmitinsäure usw.

Es ist weiterhin vorteilhaft, den erfindungsgemäßen Zusammensetzungen entsprechende wasser- und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester, Benzophenon-Derivate wie 2-Hydroxy-4-methoxybenzophenon; 3-Benzylidencampher-Derivate wie 3-Benzylidencampher.

Bevorzugte öllösliche UV-Filter sind Benzophenone-3, Butyl-Methoxybenzoylmethane, Octyl Methoxycinnamate, Octyl salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenylbenzimidazol-5-sulfonsäure.

Zu UVA-Filtern gehören Dibenzoylmethan-Derivate wie 1-Phenyl-4-(4'-isopropylphenyl)propan-1,3-dion.

Das Zusammenwirken der Hauptbestandteile des erfindungsgemäßen Kosmetikums ist noch nicht aufgeklärt. Es wurde bisher gefunden, daß die an sich bereits gute Aufhellungswirkung einer Kombination von Arbutin mit Glycyrrhiza glabra noch gesteigert wird durch den Zusatz von geringen Mengen an Astralagus-extrakt. Eine weitere Steigerung zeigt sich bei Einsatz eines Vorgemisches (Pre-Mix) von Glycyrrhiza glabra und Aspergillusferment, wobei der Aspergillusgehalt für sich allein nur im Bereich von 0,001 bis 0,1 Gew-% liegt.

Die Erfindung soll nachstehend durch Beispiele näher erläutert werden. Alle Angaben erfolgen in Gewichtsprozent, sofern nichts anderes angegeben ist.

| **Hautaufhellungscreme I und II** | Beispiel 1 | Beispiel 2 |
|---|---|---|
| **Phase A** | | |
| Wasser | ad 100 | ad 100 |
| Glycerin | 3,0 | 3,0 |
| Panthenol | 0,5 | 0,5 |
| Farbkörper weiß | 1,0 | 0,5 |
| Arbutin | 0,01 | 0,1 |
| Perlenextrakt | 0,01 | 0,015 |
| (Margaritifera calcus) | | |
| Tetranatrium EDTA | 0,1 | 0,1 |

| **Phase B** | | |
|---|---|---|
| Steareth-2 | 2,5 | 2,5 |
| Steareth-21 | 1,8 | 1,9 |
| Hydrogenated Polyisobutene & | | |
| Tocopherol | 2,0 | 2,0 |
| C12-15 Alkyl Benzoate | 1,3 | 1,3 |
| Caprylic/Capric Triglyceride | 1,5 | 1,5 |
| Isohexadecane | 1,2 | 1,25 |
| Shea Butter | 1,0 | 1,0 |
| Petrolatum | 1,2 | 1,3 |
| Cetearyl Alcohol | 1,0 | 1,0 |
| Dimethicone | 1,0 | 1,0 |
| Konservierungsmittel | 1,0 | 0,8 |

| **Phase C** | | |
|---|---|---|
| Cyclomethicone | 1,0 | 1,0 |
| Triethanolamin | 0,4 | 0,4 |
| Glycyrrhiza glabra Extrakt | 0,025 | 0,05 |
| Aspergillus Ferment | 0,005 | 0,007 |
| Astragalus Extrakt | 0,003 | 0,008 |
| Bitter Cherry Extrakt & | | |
| Malpighia punicifolia- | | |
| Fruchtextrakt | 0,01 | - |
| Parfüm | 0,3 | 0,4 |

Die separat hergestellten Phasen A und B wurden vermischt und bei etwa 40 °C homogenisiert. Bei 50 °C wurde die Phase C hergestellt und mit den beiden zuvor vermischten Phasen zugegeben.

| **Hautaufhellungslotion I und II** | Beispiel 3 | Beispiel 4 |
|---|---|---|
| Wasser | ad 100 | ad 100 |
| Glycerin | 2,0 | 2,2 |
| Farbkörper weiß | 1,0 | 1,0 |
| Arbutin | 0,01 | 0,08 |
| Perlenextrakt | 0,01 | 0,02 |
| (Margaritifera calcus) | | |
| Tetranatrium EDTA | 0,1 | 0,1 |
| Steareth-2 | 2,3 | 2,3 |
| Steareth-21 | 1,5 | 1,5 |
| Hydrogenated Polyisobutene & Tocopherol | 1,5 | 1,5 |
| Caprylic/Capric Triglyceride | 5,5 | 5,5 |
| Isohexadecane | 1,2 | 1,2 |
| Ethylhexyl Methoxycinnamate | 2,0 | 2,0 |
| Phenoxyethanol | 0,3 | 0,3 |
| Shea Butter | 0,5 | 0,6 |
| Cetearyl Alcohol | 1,0 | 1,0 |
| Acrylates/C10-30 Alkyl Acrylate Crosspolymer | 0,2 | 0,2 |
| Konservierungsmittel | 1,0 | 0,5 |
| Cyclomethicone | 1,5 | 1,4 |
| Triethanolamin | 0,2 | 0,2 |
| Glycyrrhiza glabra Extrakt | 0,1 | 0,12 |
| Aspergillus Ferment | 0,01 | 0,014 |
| Astragalus Extrakt | 0,003 | 0,009 |
| Bitter Cherry Extrakt & Malpighia punicifolia-Fruchtextrakt | 0,01 | - |
| Parfüm | 0,3 | 0,25 |

### Beispiel 5 Vergleichsbeispiel

Es wurden Untersuchungen zur Hautaufhellung nach einem Whitening-Test vorgenommen. 18 gesunde, weibliche, freiwillige Teilnehmer der Studie mit pigmentierten Flecken auf den Handrücken wurden in den Test einbezogen. Die Studienteilnehmerinnen nahmen keinerlei Medikamente und war dermatologisch unauffällig. Das Auftragen der eingesetzten Creme erfolgt für 8 Wochen zweimal täglich (morgens und abends). Keine direkte Sonneneinstrahlung und kein Waschen der Hände bis 4 Stunden nach dem Auftragen wird den Teilnehmern vorgegeben.

Zwei markierte Flächen mit Pigmentierungen auf der unbehandelten Hand und auf der behandelten Hand wurden ausgewählt. Die Messungen daran wurden zweimal wiederholt. Die Messungen erfolgten mit einem Chromamometer. Dieses Colorimeter hat eine pulsierende Xenon-Lichtbogenlampe und emittiert intensives weißes Licht, das das gesamte sichtbare Spektrum abdeckt. Drei Photozellen-Einheiten bestimmen das reflektierte Licht. Die Messungen erfolgten mit C.I.E. D65 Beleuchtung. Die Daten werden im L*a*b*-System angezeigt, wobei die Farbe in dreidimensionalen Koordinaten mit der a⁺-Achse (rot-grün), einer b*-Achse (gelb-blau) und einer L*-Achse (Helligkeit) ausgedrückt werden. Die ergebnisse sind Angaben in Prozent Färbung, wobei der Ausgangspunkt 100% entspricht.

Probe A ist eine Placebo-Probe.
Probe B ist die Basiszusammensetzung des Beispiels 1 ohne Perlenextrakt, Aspergillusferment und Astralagus-Extrakt.
Die Probe C enthält die gleiche Zusammensetzung wie Beispiel 1 ohne Aspergillusferment.
Die Probe D enthält die Zusammensetzung wie im Beispiel 1, wobei Glycyrrhiza glabra und Aspergillusferment als Pre-Mix eingebracht werden.

| | Probe A | Probe B | Probe C | Probe D |
|---|---|---|---|---|
| vor der Behandlung | 100% | 100% | 100% | 100% |
| nach 2 Wochen | 100% | 99% | 96% | 95% |
| nach 4 Wochen | 100% | 97% | 95% | 93% |
| nach 6 Wochen | 100% | 97% | 92% | 90% |
| nach 8 Wochen | 100% | 96% | 89% | 87% |

Aus dem Ergebnis ist deutlich ersichtlich, daß die Probe B, die Arbutin enthält, zwar eine bestimmte Aufhellung um bis zu 5 % über 8 Wochen bewirkt, diese Werte jedoch deutlich von denen der Proben C und D mit den erfindungsgemäßen Zusätzen übertroffen werden.

## Patentansprüche

1. Hautaufhellendes Kosmetikum, **gekennzeichnet durch** einen Gehalt an 0,01 bis 0,5 Gew-% Arbutin,
0,01 bis 0,05 Gew-% eines Perlenextraktes,
0,05 bis 0,6 Gew-% eines Extraktgemisches von Glycyrrhiza glabra und Aspergillusferment und
0,001 bis 0,1 Gew-% eines Extraktes von Astragalus,
sowie weiteren kosmetischen Hilfsstoffen, Trägerstoffen und Wirkstoffen bis 100 Gew-%, jeweils bezogen auf das Gesamtgewicht des Kosmetikums.

2. Kosmetikum nach Anspruch 1, **dadurch gekennzeichnet, daß** es 0,1 bis 5,0 Gew-% eines Sonnenschutzfilters enthält.

3. Kosmetikum nach Anspruch 2, **dadurch gekennzeichnet, daß** der Sonnenschutzfilter ein organischer öllöslicher Filter ist, ausgewählt unter Benzophenone-3, Octyl Methoxycinnamate, Octylsalicylate, Homosalate und 4-Methylbenzyliden Camphor.

4. Kosmetikum nach Anspruch 1, **dadurch gekennzeichnet, daß** das Verhältnis von Glycyrrhiza-Extrakt und Aspergillusferment im Bereich von 30:1 bis 5:1 liegt.

5. Kosmetikum nach Anspruch 1, **dadurch gekennzeichnet, daß** der Gehalt von Arbutin im Bereich von 0,01 bis 0,1 Gew-% liegt.

6. Kosmetikum nach Anspruch 1, **dadurch gekennzeichnet, daß** der Gehalt des Extraktes von Glycyrrhiza glabra im Bereich von 0,01 bis 0,2 Gew-% liegt.

7. Kosmetikum nach Anspruch 1, **dadurch gekennzeichnet, daß** der Gehalt von Astragalusextrakt im Bereich von 0,001 bis 0,01 Gew-% liegt.

## Claims

1. A skin-whitening cosmetic comprising
0.01 to 0.5% by weight of arbutin,
0.01 to 0.05% by weight of a pearl extract,
0.05 to 0.6% by weight of an extract mixture of Glycyrrhiza glabra and Aspergillus ferment, and
0.001 to 0.1% by weight of an Astragalus extract
and other cosmetic adjuvants, excipients and agents up to 100% by weight, all percentages relating to the cosmetic's total weight.

2. A cosmetic according to Claim 1, wherein said cosmetic contains 0.1 to 5.0% by weight of a sunscreen.

3. A cosmetic according to Claim 2, wherein said sunscreen is an organic, oil-soluble filter selected from among benzophenone-3, octyl methoxycinnamate, octyl salicylate, homosalate and 4-methylbenzylidene camphor.

4. A cosmetic according to Claim 1, wherein the ratio of Glycyrrhiza extract to Aspergillus ferment ranges between 30:1 and 5:1.

5. A cosmetic according to Claim 1, wherein arbutin in contained in an amount ranging between 0.01 and 0.1% by weight.

6. A cosmetic according to Claim 1, wherein the extract of Glycyrrhiza glabra is contained in an amount ranging between 0.01 and 0.2% by weight.

7. A cosmetic according to Claim 1, wherein Astragalus extract is contained in an amount ranging between 0.001 and 0.01% by weight.

## Revendications

1. Produit cosmétique à effet éclaircissant, **caractérisé par** une teneur de
0,01 à 0,5 % en poids d'arbutine,
0,01 à 0,05 % en poids d'un extrait de nacre,
0,05 à 0,6 % en poids d'un mélange d'extrait de *Glycyrrhiza glabra* et de ferment d'*Aspergillus* et
0,001 à 0,1 % en poids d'un extrait d'astragale,
ainsi que d'autres excipients, supports et principes actifs cosmétiques pour faire 100 % en poids, respectivement par rapport au poids total du produit cosmétique.

2. Produit cosmétique selon la revendication 1, **caractérisé en ce qu'**il contient 0,1 à 5,0 % en poids d'un filtre photoprotecteur.

3. Produit cosmétique selon la revendication 2, **caractérisé en ce que** le filtre photoprotecteur est un filtre liposoluble organique, choisi parmi les benzophénones-3, les octyl méthoxycinnamates, les octyl salicylates, les homosalates et le 4-méthylbenzylidène camphre.

4. Produit cosmétique selon la revendication 1, **caractérisé en ce que** le rapport extrait de *Glycyrrhiza*/ferment d'*Aspergillus* se situe dans la plage de 30/1 à 5/1.

5. Produit cosmétique selon la revendication 1, **caractérisé en ce que** la teneur en arbutine se situe dans la plage allant de 0,01 à 0,1 % en poids.

6. Produit cosmétique selon la revendication 1, **caractérisé en ce que** la teneur en extrait de *Glycyrrhiza glabra* se situe dans la plage allant de 0,01 à 0,2 % en poids.

7. Produit cosmétique selon la revendication 1, **caractérisé en ce que** la teneur en extrait d'astragale se situe dans la plage de 0,001 à 0,01 % en poids.
